# EUROPÄISCHE PATENTSCHRIFT

(11) **EP 1 051 409 B1**
(45) Veröffentlichungstag und Bekanntmachung des Hinweises auf die Patenterteilung: **13.08.2003**
(21) Anmeldenummer: 99907392.7
(22) Anmeldetag: 20.01.1999
(51) Int. Cl.: C07D 251/60

(54) **VERFAHREN ZUM ABKÜHLEN VON MELAMIN**
METHOD FOR COOLING MELAMINE
PROCEDE DE REFROIDISSEMENT DE MELAMINE

(30) Priorität: 30.01.1998 AT 15998
(43) Veröffentlichungstag der Anmeldung: 15.11.2000
(73) Patentinhaber: Agrolinz Melamin GmbH, A-4021 Linz (AT)
(72) Erfinder: COUFAL, Gerhard, A-4060 Leonding (AT)
(74) Vertreter: Gross, Felix
(86) Internationale Anmeldenummer: EP9900353
(87) Internationale Veröffentlichungsnummer: WO99038852

(56) Entgegenhaltungen:
- WO-A-95/01345
- US-A- 4 565 867

## Beschreibung

Die Anmeldung betrifft ein Verfahren zum Abkühlen von flüssigem Melamin durch Vermischen mit festem Melamin.

Aus der Literatur ist bereits eine Vielzahl von Verfahren zur Herstellung von Melamin bekannt (Ullmann's Encyclopedia of Industrial Chemistry, 5^{th} Edition, Vol. A-16, pp 174-179). Alle technisch wesentlichen Verfahren gehen von Harnstoff aus, der entweder bei hohem Druck und nicht katalytisch oder bei niedrigem Druck und unter Verwendung eines Katalysators zu Melamin, Ammoniak und CO₂ umgesetzt wird.

Bei den Niederdruckverfahren fällt gasförmiges Melamin an, bei den Hochdruckverfahren im Wesentlichen flüssiges Melamin. Vorhandenes gasförmiges Melamin wird zusammen mit den Offgasen CO₂ und NH₃ durch eine Harnstoffschmelze geleitet, wobei die Offgase gekühlt, das Melamin im Harnstoff gelöst und der Harnstoff erwärmt und dem Reaktor zur Melaminsynthese zugeführt wird. Gasförmiges Melamin wird auch nach dem Hochdruckprozeß von WO95/01345 (Kemira), erzeugt, wobei die zuletzt erhaltene Melaminschmelze verdampft.

Ein großes Problem beim Abkühlen und Verfestigen von flüssigern Melamin besteht darin, daß eine Temperaturdifferenz von über 300 °C durchschritten werden muß, und sich dabei Nebenprodukte bilden können. Eine gängige Methode zur Abkühlung ist das Abschrecken ("quenchen") mit Wasser oder mit Wasserdampf, wobei meist umkristallisiert werden muß, um die verschiedenen Nebenprodukte zu entfernen. Wird mit Gas, etwa mit gasförmigem Ammoniak gequencht, müssen sehr große Mengen an Gas eingesetzt und im Kreislauf geführt werden. Wird mit flüssigem Ammoniak, etwa nach US-4,565,867 gequencht, wird zwar die Verdampfungswärme des Ammoniaks zur Kühlung ausgenützt, es müssen jedoch ebenfalls große Gasmengen im Kreislauf geführt und immer wieder komprimiert werden.

Unerwarteterweise konnte nun ein einfaches Verfahren gefunden werden, bei dem die Bildung von Nebenprodukten hintangehalten wird, und bei dem keine großen Gasmengen im Kreislauf geführt und wieder verdichtet werden müssen.

Gegenstand der Erfindung ist demnach ein Verfahren zum Abkühlen von flüssigem Melamin durch Vermischen mit festem Melamin oder mit festen Inertstoffen oder mit einem Gemisch aus festem Melamin und festen Inertstoffen.

Als feste Inertstoffe kommen bevorzugt Metall- oder Glaspartikel in Frage, beispielsweise Kugeln oder Stäbchen aus Stahl, insbesondere Edelstahl, Stahl- oder Titanlegierungen. Es ist auch möglich zusätzlich durch Zufuhr von kaltem flüssigem oder gasförmigem NH₃ bzw. durch zusätzliche Kühlelemente und Wärmetauscher zu kühlen.

Zur Vermischung des flüssigen Melamins mit festem Melamin kann sowohl festes Melamin in flüssiges Melamin als auch flüssiges Melamin in festes Melamin eingebracht werden, oder die Reaktionspartner treffen in einem Entspannungs- und Abschreckungsgefäß ("Quencher") aufeinander. Dabei ist es bevorzugt, wenn das flüssige Melamin beim Vermischen entspannt wird. Es erweist sich als vorteilhaft, während des Vermischens zusätzliches NH₃ zuzuführen. Die Abkühlung des Melamins erfolgt bevorzugt bis unterhalb des Schmelzpunktes von Melamin.

Das abzukühlende flüssige Melamin steht unter einem gewissen Ammoniak-Druck, etwa von 1 bis 1000 bar. Da flüssiges Melamin in Abhängigkeit von Druck und Temperatur Nebenprodukte wie z.B. Melam, Melem, Melon, Ureidomelamin, Ammelin oder Ammelid enthält, bzw. zur Abspaltung von NH₃ neigt, liegt es vorzugsweise unter Ammoniak-Druck vor. Je höher dieser Ammoniak-Druck ist, umso geringer ist der Gehalt an Nebenprodukten. Je nach durchgeführten Melamin-Herstellungsverfahren liegt das zu kühlende flüssige Melamin vorteilhafterweise unter einem Ammoniak-Druck von etwa 40 bis 1000 bar, bevorzugt von etwa 40 bis 400 bar, besonders bevorzugt unter einem Druck von etwa 60 bis 300 bar vor.

Das Abkühlen von flüssigem Melamin kann beispielsweise so erfolgen, daß festes Melamin in flüssiges Melamin, das unter einem bestimmten Ammoniak-Druck steht, eingebracht wird. Das feste Melamin erwärmt sich beim Einbringen und Mischen mit der Schmelze, während die Schmelze sich abkühlt. Der Ammoniak-Druck unter dem sich die Schmelze befindet, kann dabei gleichbleiben, erhöht werden, oder vermindert werden. Bevorzugt wird er in einem kontinuierlichen Verfahren etwa gleichbleiben.

Die Temperatur der Schmelze, bzw. der entstandenen Mischung kann dabei gegebenenfalls unter Zuhilfenahme von zusätzlicher Kühlung bis unterhalb des Festpunktes von Melamin abgesenkt werden, sodaß auf schonende Weise reines und festes Melamin entsteht. Gegebenenfalls verweilt das gebildete feste Melamin noch eine bestimmte Zeit unter Ammoniakdruck, anschließend wird entspannt.

Es ist aber auch möglich, die Temperatur des abzukühlenden flüssigen Melamins nur bis zu oder bis knapp oberhalb des vom jeweiligen Ammoniak-Druck abhängigen Festpunktes des Melamins zu senken, wobei es möglich ist, dem festen Melamin auch Ammoniak, etwa in flüssigem, gasförmigen oder überkritischem Zustand beizufügen, um das flüssige Melamin, das bei niedrigerer Temperatur mehr Ammoniak aufnehmen kann, mit Ammoniak zu sättigen. Diese Vorgangsweise kann beispielsweise auch dann verwendet werden, wenn die flüssige, mit NH₃ gesättigte Melaminschmelze anschließend etwa gemäß WO 97/20826 entspannt und verfestigt werden soll.

Die bevorzugte Möglichkeit zur Abkühlung von flüssigem Melamin mit festem Melamin besteht darin, es bis unterhalb des Festpunktes zu kühlen.

Dabei ist es möglich, die Mischungspartner unter Beibehaltung des vorhandenen Druckes, mit nachfolgender Druckerhöhung oder unter Druckverminderung zu mischen. Bevorzugt erfolgt die Mischung unter Druckverminderung.

Es ist möglich, festes Melamin in flüssiges Melamin oder flüssiges in festes Melamin einzutragen, oder beide Mischungspartner gleichzeitig in einen Quencher einzutragen.

Nach einer bevorzugten Ausführungsform wird festes Melamin in einem Behälter vorgelegt und flüssiges Melamin eingetragen, bevorzugt unter Druckverminderung.

Besonders bevorzugt ist es, das Vermischen in einem Wirbelbett durchzuführen.

Zu Beginn der Reaktion wird festes Melamin oder artfremdes Material in Form von festen Inertstoffen oder ein Gemisch aus festem Melamin und festen Inertstoffen in den Wirbelschichtreaktor eingetragen und zum Aufbau des Wirbelbettes verwendet. Als feste Inerstoffe werden bevorzugt Wirbelkörper aus Metallen oder Glas, beispielsweise Kugeln oder Stäbchen aus Stahl, insbesondere Edelstahl, Stahl- oder Titanlegierungen eingesetzt. Das Wirbelbett wird mit einem Gas, bevorzugt mit Ammoniak, aufrechterhalten. Die Temperatur im Wirbelschichtreaktor liegt unterhalb des Schmelzpunktes von Melamin. Flüssiges Melamin wird eingedüst. Das fein verteilte, flüssige Melamin bildet eine Schicht über den festen Melamin- oder Inertstoffpartikeln, läßt diese wachsen und wird fest. Durch die Bewegung und Reibung der Partikel im Wirbelbett wird laufend Melamin von den Partikeln abgerieben bzw. abgeschlagen. Die größeren und damit schwereren Melaminteilchen werden etwa mit Hilfe eines Zyklons ausgetragen, sobald sie eine bestimmte gewünschte Korngröße erreicht haben. Einerseits kann festes kaltes Melamin zu einem geringeren Anteil laufend zugeführt werden, damit sich an ihm das flüssige Melamin abscheiden und verfestigen kann, andererseits bilden sich je nach Ausführungsart des Wirbelschichtreaktors und je nach den in der Wirbelschicht herrschenden sonstigen Bedingungen bereits im Gasraum feste Melaminpartikel, die als Kristallisationskeime dienen und sich mit flüssigem Melamin überziehen, das dann ebenfalls erstarrt. In diesem Fall muß kein oder fast kein festes Melamin von außen zugeführt werden.

Die Kühlung der festen Melamin- und Inertstoffpartikel im Wirbelbett und damit die Einstellung der gewünschten Temperatur im Wirbelbett kann auf mehrfache Weise erfolgen, beispielsweise durch eingebaute Kühlelemente, durch Zufuhr von festem kaltem Melamin, durch gegebenenfalls ausgeschleuste und nach externer Kühlung wieder in das Wirbelbett rückgeführte Inertpartikel, durch Zufuhr von kaltem flüssigem oder gasförmigem NH₃, durch die Temperatur und Menge des Gasstromes, mit dem die Wirbelschicht aufrechterhalten wird, und durch die Verdampfungsenthalpie des im flüssigen Melamin enthaltenen Ammoniaks.

Ein Teil dieses Ammoniaks wird, zur Kühlung und zur Aufrechterhaltung des Wirbelbettes, im Kreislauf geführt. Das Ammoniak wird bevorzugterweise vor der Rückführung in das Wirbelbett gekühlt und gegebenenfalls verflüssigt. Der andere Teil des freiwerdenden Ammoniaks kann je nach vorhandenem Druck im Wirbelbett gasförmig oder verflüssigt in den Melamin/Harnstoffprozeß rückgeführt werden. Hier zeigt sich ein besonderer Vorteil des erfindungsgemäßen Verfahrens, da kein zusätzliches, nicht aus dem Melamin/Harnstoffprozeß stammendes Gas, bzw. Ammoniak zur Aufrechterhaltung des Wirbelbettes notwendig ist.

Die im Wirbelbett vorhandene und aufrechterhaltene Temperatur kann je nach gewählter Verfahrensweise in einem großen Bereich zwischen Raumtemperatur und bis knapp unterhalb des druckabhängigen Schmelzpunktes von Melamin schwanken. Sie beträgt beispielsweise etwa 100 bis etwa 340°C, bevorzugt etwa 200 bis etwa 340 °C, besonders bevorzugt etwa 280 bis etwa 320°C.

Der im Wirbelschichtreaktor vorhandene Druck kann je nach gewählter Verfahrensweise ebenfalls in einem großen Bereich schwanken. Er kann von zwischen etwas über 1 bar bis knapp unterhalb des Druckes der zu kühlenden Melaminschmelze betragen.

Üblicherweise beträgt der Druck im Wirbelschichtreaktor zwischen etwa 1,5 und etwa 100 bar, bevorzugt zwischen etwa 1,5 bar und 50 bar, besonders bevorzugt zwischen etwa 5 bis 25 bar. Über einem Druck von über etwa 13 bar kann das überschüssige NH₃-Gas leicht verflüssigt und in die Harnstoff- und Melaminsynthese rückgeführt werden.

Der NH₃-Druck über der zu kühlenden Melaminschmelze kann ebenfalls in einem großen Bereich variieren. Häufig liegt er beim Druck der im Reaktor durchgeführten Melaminsynthese. Er kann jedoch wesentlich höher liegen, wenn der Melaminsynthese ein "Aging" nachgeschaltet ist. Der Druck kann demnach bis zu 1000 bar oder bis zu den ökonomisch und materialmäßig sinnvollen und möglichen Grenzen betragen. Beim Eintrag der Melaminschmelze in den Wirbelschichtreaktor wird auf den dort herrschenden Druck entspannt, wobei das flüssige Melamin abgekühlt und verfestigt wird.

Grundsätzlich kann die Temperatur des zu kühlenden flüssigen Melamins in einem großen Bereich variieren. Sie liegt oberhalb des vom jeweiligen Ammoniak-Druck abhängigen Schmelzpunktes von Melamin in einem Bereich bis zu etwa 450 °C, bevorzugt bis zu etwa 370 °C, besonders bevorzugt bis zu etwa 350 °C. Je höher der Ammoniakdruck ist und je niedriger die Temperatur der Melaminschmelze ist, umso mehr Ammoniak ist im Melamin enthalten, und umso niedriger ist der Schmelzpunkt. Bei einem Ammoniakdruck von 300 bar liegt beispielsweise der Schmelzpunkt bei etwa 300 °C, bei 1 bar bei 354°C. Es ist also auch möglich, bei 300°C flüssiges Melamin, genauer gesagt, eine Mischung von flüssigem Melamin mit Ammoniak vorliegen zu haben und zu entspannen, falls der Druck hoch genug ist.

Besonders vorteilhaft ist es, bei einer Temperatur, die nicht wesentlich über dem jeweiligen Schmelzpunkt des Melamins liegt, zu entspannen, und mit dem festen Melamin zu mischen. Diese Abkühlung bis knapp oberhalb des Schmelzpunktes des Melamins erfolgt bevorzugt durch Zufuhr von kaltem flüssigem oder gasförmigem, bzw. von überkritischem Ammoniak. Das im flüssigen Melamin enthaltene Ammoniak trägt beim nachfolgenden Entspannen ebenfalls zur Abkühlung bei und wirkt der beim Erstarren des Melamins freiwerdenden Schmelzenthalpie entgegen.

Falls festes Melamin zugeführt wird, kann die Temperatur des festen Melamins bei jedem beliebigen Wert unterhalb des Schmelzpunktes von Melamin liegen, wobei eine größere Temperaturdifferenz zwischen festem und zu kühlendem, flüssigem Melamin einen größeren Kühleffekt hat. Vorteilhafterweise können anfallende Melamin-Feinanteile in den Wirbelschichtreaktor rückgeführt werden, und dienen dort als Kristallisationskeime.

Eine weitere Möglichkeit der Temperatursteuerung besteht im Eindüsen von flüssigem Ammoniak.

Die Temperatur des auszutragenden festen Melamins kann jeden Wert unterhalb des Schmelzpunktes von Melamin betragen, bevorzugt liegt sie unterhalb von etwa 320°C, besonders bevorzugt unterhalb von etwa 300°C. Das feste Melamin, das gewünschtenfalls noch einer Wärmebehandlung unter Ammoniak-Druck (Tempern) unterzogen werden kann, wird dann auf beliebige Weise weiter entspannt und bis auf Raumtemperatur gekühlt. Beim Tempern wird das bereits feste Melamin unterhalb des vom jeweiligen Ammoniak-Druck abhängigen Schmelzpunktes verweilen gelassen, beispielsweise für etwa 1 min bis 20 h, unter einem Ammoniak-Druck von etwa 5 bis 1000 bar bei einer Temperatur von etwa 100 °C, bevorzugt von etwa 200 °C, bis unterhalb des vom jeweiligen Ammoniak-Druck abhängigen Schmelzpunktes.

Das erfindungsgemäße Verfahren wird bevorzugt im Anschluß an eine Melaminsynthese aus Harnstoff, besonders bevorzugt im Anschluß an eine Melaminsynthese unter Druck, durchgeführt.

### Beispiel:

In einer Pilotanlage wurde das vom Reaktor einer Produktionsanlage abgezogene Melamin von den Reaktionsgasen (Offgasen) CO₂/NH₃ in einem Separator getrennt, in einem nachgeschalteten Reaktionsgefäß mit 100 kg Ammoniak/h bei einem Druck von 100 bar gestrippt und dann in einen Agingbehälter geleitet. Bei einem NH₃-Druck von 250 bar und einer Temperatur von 330 °C wurde die Melaminschmelze mit NH₃ gesättigt und eine Stunde verweilen gelassen. Vom Agingbehälter wurden dann ca. 11 kg Melaminschmelze/h in ein Melaminwirbelbett gesprüht. Das Wirbelbett wurde mit NH₃-Gas aufrechterhalten und bei einem Druck von 25 bar und einer Temperatur von 300 °C betrieben. Festes Melamin wurde ausgeschleust, entspannt und auf Raumtemperatur gekühlt.

Reinheit: 99,8 Gew.% Melamin.

## Patentansprüche

1. Verfahren zum Abkühlen von flüssigem Melamin durch Vermischen mit festem Melamin oder mit festen Inertstoffen oder mit einem Gemisch aus festem Melamin und festen Inertstoffen.

2. Verfahren nach Anspruch 1, **dadurch gekennzeichnet, daß** das flüssige Melamin unter einem NH₃-Druck von 1 - 1000 bar steht.

3. Verfahren nach Anspruch 1, **dadurch gekennzeichnet, daß** während des Vermischens NH₃ zugeführt wird.

4. Verfahren nach Anspruch 1, **dadurch gekennzeichnet, daß** das Abkühlen bis unterhalb des Schmelzpunktes von Melamin erfolgt.

5. Verfahren nach Anspruch 1, **dadurch gekennzeichnet, daß** das flüssige Melamin beim Vermischen entspannt wird.

6. Verfahren nach Anspruch 1, **dadurch gekennzeichnet, daß** das flüssige Melamin vor dem Abkühlen mit NH₃ gesättigt ist.

7. Verfahren nach Anspruch 1, **dadurch gekennzeichnet, daß** das flüssige Melamin beim Vermischen entspannt und bis unterhalb des Schmelzpunktes vom Melamin abgekühlt wird.

8. Verfahren nach Anspruch 1, **dadurch gekennzeichnet, daß** die festen Inertstoffe aus Metall- oder Glaspartikeln bestehen.

9. Verfahren nach einem der Ansprüche 1 - 8, **dadurch gekennzeichnet, daß** das Vermischen in einem Wirbelbett erfolgt.

10. Verfahren nach Anspruch 9, **dadurch gekennzeichnet, daß** die Temperatur im Wirbelbett etwa 100 bis etwa 340 °C beträgt.

11. Verfahren nach Anspruch 9, **dadurch gekennzeichnet, daß** der Druck im Wirbelbett etwa 1,5 bis etwa 100 bar beträgt.

12. Verfahren nach Anspruch 9, **dadurch gekennzeichnet, daß** das Wirbelbett aus festem Melamin aufgebaut ist.

13. Verfahren nach Anspruch 9, **dadurch gekennzeichnet, daß** das Wirbelbett aus festem Melamin und festen Inertstoffen aufgebaut ist.

14. Verfahren nach Anspruch 9, **dadurch gekennzeichnet, daß** das Wirbelbett mit einem Gas, bevorzugt mit Ammoniak aufrechterhalten wird.

15. Verfahren nach einem der Ansprüche 1 bis 14, **dadurch gekennzeichnet, daß** das flüssige Melamin bis unterhalb des Schmelzpunktes abgekühlt und anschließend für etwa 1 min bis 20 h unter einem Ammoniak-Druck von etwa 5 bis 1000 bar bei einer Temperatur von etwa 100 °C bis unterhalb des Schmelzpunktes verweilen gelassen wird.

16. Verfahren nach einem der Ansprüche 1 bis 15, **dadurch gekennzeichnet, daß** es im Anschluß an eine unter Druck durchgeführte Melaminsynthese aus Harnstoff durchgeführt wird.

## Claims

1. Process for the cooling of liquid melamine by mixing with solid melamine or with solid inert substances or with a mixture of solid melamine and solid inert substances.

2. Process according to Claim 1, **characterised in that** the liquid melamine is under an NH₃ pressure of 1-1000 bars.

3. Process according to Claim 1, **characterised in that** NH₃ is introduced during the mixing.

4. Process according to Claim 1, **characterised in that** the cooling takes place to below the melting point of melamine.

5. Process according to Claim 1, **characterised in that** the liquid melamine is depressurised during the mixing.

6. Process according to Claim 1, **characterised in that** the liquid melamine is saturated with NH₃ before the cooling.

7. Process according to Claim 1, **characterised in that** the liquid melamine is depressurised during the mixing and cooled to below the melting point of melamine.

8. Process according to Claim 1, **characterised in that** the solid inert substances consist of metal or glass particles.

9. Process according to one of Claims 1-8, **characterised in that** the mixing takes place in a fluidised bed.

10. Process according to Claim 9, **characterised in that** the temperature in the fluidised bed is about 100 to about 340°C.

11. Process according to Claim 9, **characterised in that** the pressure in the fluidised bed is about 1.5 to about 100 bars.

12. Process according to Claim 9, **characterised in that** the fluidised bed is constituted of solid melamine.

13. Process according to Claim 9, **characterised in that** the fluidised bed is constituted of solid melamine and solid inert substances.

14. Process according to Claim 9, **characterised in that** the fluidised bed is maintained with a gas, preferably with ammonia.

15. Process according to one of Claims 1 to 14, **characterised in that** the liquid melamine is cooled to below the melting point and then allowed to remain for about 1 min to 20 hrs under an ammonia pressure of about 5 to 1000 bars at a temperature of about 100°C to below the melting point.

16. Process according to one of Claims 1 to 15, **characterised in that** it is performed following a melamine synthesis from urea performed under pressure.

## Revendications

1. Procédé de refroidissement de mélamine liquide par mélange avec de la mélamine solide ou avec des substances inertes solides ou avec un mélange de mélamine solide et de substances inertes solides.

2. Procédé selon la revendication 1, **caractérisé en ce que** la mélamine liquide se trouve sous une pression de NH₃ de 1 à 1000 bar.

3. Procédé selon la revendication 1, **caractérisé en ce que** l'on amène du NH₃ pendant le mélange.

4. Procédé selon la revendication 1, **caractérisé en ce que** l'on effectue le refroidissement jusqu'à une température inférieure au point de fusion de la mélamine.

5. Procédé selon la revendication 1, **caractérisé en ce que** l'on détend la mélamine liquide lors du mélange.

6. Procédé selon la revendication 1, **caractérisé en ce que** la mélamine liquide est saturée de NH₃ avant le refroidissement.

7. Procédé selon la revendication 1, **caractérisé en ce que** l'on détend la mélamine liquide lors du mélange et on la refroidit jusqu'à une température inférieure au point de fusion de la mélamine.

8. Procédé selon la revendication 1, **caractérisé en ce que** les substances inertes solides sont constituées de particules de métal ou de verre.

9. Procédé selon l'une des revendications 1 à 8, **caractérisé en ce que** le mélange s'effectue dans un lit fluidisé.

10. Procédé selon la revendication 9, **caractérisé en ce que** la température dans le lit fluidisé est d'environ 100 à environ 340°C.

11. Procédé selon la revendication 9, **caractérisé en ce que** la pression dans le lit fluidisé est d'environ 1,5 à environ 100 bar.

12. Procédé selon la revendication 9, **caractérisé en ce que** le lit fluidisé est composé de mélamine solide.

13. Procédé selon la revendication 9, **caractérisé en ce que** le lit fluidisé est composé de mélamine solide et de substances inertes solides.

14. Procédé selon la revendication 9, **caractérisé en ce que** le lit fluidisé est maintenu par un gaz, de préférence de l'ammoniac.

15. Procédé selon l'une des revendications 1 à 14, **caractérisé en ce que** l'on refroidit la mélamine liquide jusqu'à une température inférieure au point de fusion, puis on la laisse séjourner pendant environ 1 minute à 20 heures sous une pression d'ammoniac d'environ 5 à 1000 bar à une température allant d'environ 100°C à une température inférieure au point de fusion.

16. Procédé selon l'une des revendications 1 à 15, **caractérisé en ce qu'**on l'effectue à la suite d'une synthèse de mélamine à partir d'urée effectuée sous pression.
